# EUROPEAN PATENT APPLICATION

(11) **EP 2 426 102 A1**
(43) Date of publication of application: **07.03.2012**
(21) Application number: 10769850.8
(22) Date of filing: 23.04.2010
(51) Int. Cl.: C07C 51/62, C07C 63/72, C07B 61/00

(54) **PROCESS FOR PRODUCING PHTHALIC ACID COMPOUND INCLUDING CHLORINATED AROMATIC RING**

(30) Priority: 28.04.2009 JP 2009108931
(71) Applicant: Sumitomo Chemical Company, Limited, Tokyo 104-8260 (JP)
(72) Inventor: SOUDA, Hiroshi, Oita-shi Oita 870-0025 (JP); HAGIYA, Koji, Ibaraki-shi Osaka 567-0833 (JP)
(74) Representative: Duckworth, Timothy John
(86) International application number: PCT/JP2010/057731
(87) International publication number: WO 2010/126152

(57) **Abstract**

Provided is a process for producing a phthalic acid compound whose aromatic ring has been chlorinated, the process reacting a phthalic acid compound and chlorine in a mixture of chlorosulfonic acid and thionyl chloride in the presence of an iodine compound.

## Description

### TECHNICAL FIELD

The present invention relates to a process for producing a phthalic acid compound whose aromatic ring has been chlorinated.

### BACKGROUND ART

A phthalic acid compound whose aromatic ring has been chlorinated is useful as flame retardant polymers, organic pigments, pharmaceutical and agrochemical ingredients, electronic materials, synthetic intermediates thereof, and the like (See e.g. US Pat 4,001,179).

As methods for producing a phthalic acid compound whose aromatic ring has been chlorinated, for example, a method of reacting phthalic anhydride and chlorine in chlorosulfonic acid in the presence of iodine is described in Japanese Unexamined Patent Application Publication No. S61-118378, and a method of reacting terephthaloyl chloride and chlorine in a mixed solvent of chlorosulfonic acid and carbon tetrachloride in the presence of iodine is described in Japanese Unexamined Patent Application No. S58-157727.

### DISCLOSURE OF THE INVENTION

The present application relates to a novel process for producing a phthalic acid compound whose aromatic ring has been chlorinated.

The present application relates to the following invention.
[1] A method for producing a phthalic acid compound whose aromatic ring has been chlorinated, said method comprising reacting a phthalic acid compound and chlorine in the presence of an iodine compound in a mixture of chlorosulfonic acid and thionyl chloride.
[2] The production method as described in [1], wherein the phthalic acid compound is phthalic acid, isophthalic acid, terephthalic acid, a phthaloyl monohalide, an isophthaloyl monohalide, a terephthaloyl monohalide, a phthaloyl dihalide, an isophthaloyl dihalide, or a terephthaloyl dihalide.
[3] The production method as described in [1], wherein the phthalic acid compound is phthalic acid, a phthaloyl dihalide, isophthalic acid, an isophthaloyl dihalide, terephthalic acid or a terephthaloyl dihalide.
[4] The production method as described in [1], wherein the phthalic acid compound is represented by formula (1) 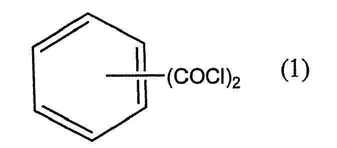 and the phthalic acid compound whose aromatic ring has been chlorinated is represented by formula (2) 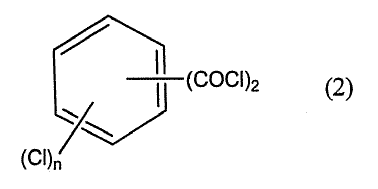 wherein n is an integer of from 1 to 4.
[5] The production method as described in [4], wherein n is 4 in formula (2).
[6] The production method as described in any one of [1] to [5], wherein the iodine compound is iodine, iodine bromide or iodine chloride.
[7] A production method for a phthalic acid compound whose aromatic ring has been chlorinated, the method comprising steps of:
   reacting a phthalic acid compound and chlorine in the presence of an iodine compound in a mixture of chlorosulfonic acid and thionyl chloride to obtain a reaction mixture; and
   isolating a mixture containing chlorosulfonic acid, thionyl chloride and the iodine compound from said reaction mixture to obtain the phthalic acid compound whose aromatic ring has been chlorinated.
[8] The production method as described in [7], wherein the phthalic acid compound whose aromatic ring has been chlorinated is represented by formula (2) 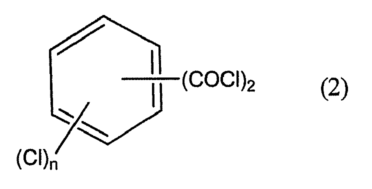 wherein n is an integer of from 1 to 4.
[9] The production method as described in [8], wherein n is 4 in formula (2).
[10] A method for producing a phthalic acid compound whose aromatic ring has been chlorinated, wherein a reaction of a phthalic acid compound and chlorine is performed in the presence of the mixture described in any one of said [7] to [9].
[11] A method for producing a phthalic acid compound whose aromatic ring has been chlorinated, the method comprising steps of:
   reacting a phthalic acid compound and chlorine in the presence of an iodine compound in a mixture of chlorosulfonic acid and thionyl chloride to obtain a reaction mixture;
   isolating a mixture containing chlorosulfonic acid, thionyl chloride and the iodine compound from said reaction mixture to obtain the phthalic acid compound whose aromatic ring has been chlorinated; and
   performing a reaction of a phthalic acid compound and chlorine in the presence of said mixture.

### MODE FOR CARRYING OUT THE INVENTION

The present invention will now be described in detail.

In the production method of the present invention, a reaction of a phthalic acid compound and chlorine is performed.

The above phthalic acid compound includes phthalic acid, isophthalic acid, terephthalic acid, acid monohalides and acid dihalides thereof and phthalic anhydride.

Haloformyl groups in the above acid monohalides and acid dihalides include a fluoroformyl group, a chloroformyl group, an iodoformyl group and a bromoformyl group.

The above acid monohalides include phthaloyl monohalides such as phthaloyl monochloride and phthaloyl monofluoride; isophthaloyl monohalides such as isophthaloyl monochloride and isophthaloyl monofluoride; and terephthaloyl monohalides such as terephthaloyl monochloride and terephthaloyl monofluoride.

The above acid dihalides include phthaloyl dihalides such as phthaloyl dichloride and phthaloyl difluoride; isophthaloyl dihalides such as isopthalic acid dichloride and isophthaloyl difluororide; and terephthaloyl dihalides such as terephthaloyl dichloride and terephthaloyl difluoride. As the above acid dihalides, an acid dichloride represented by formula (1) 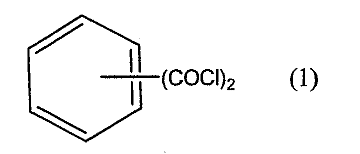 (hereinafter this compound is referred to as acid dichloride (1)) is preferred, and isophthaloyl dichloride and terephthaloyl dichloride are more preferred.

In the above phthalic acid compound, the benzene ring may be bound by 1 to 3 substituents such as a halogen atom, a nitro group, a sulfo group and a trihalomethyl group. The above halogen atom includes chlorine, fluorine, iodine, and bromine. The above trihalomethyl group includes a trichloromethyl group, a trifluoromethyl group, a triiodomethyl group, and a tribromomethyl group.

The above phthalic acid compound is preferably phthalic acid, isophthalic acid, terephthalic acid, a phthaloyl monohalide, an isophthaloyl monohalide, a terephthaloyl monohalide, a phthaloyl dihalide, an isophthaloyl dihalide and a terephthaloyl dihalide, more preferably phthalic acid, a phthaloyl dihalide, isophthalic acid, an isophthaloyl dihalide, terephthalic acid and a terephthaloyl dihalide, further preferably isophthalic acid, an isophthaloyl dihalide, terephthalic acid and a terephthaloyl dihalide, particularly preferably isophthalic acid, isophthaloyl dichloride, terephthalic acid and terephthaloyl dichloride.

The phthalic acid compounds can be produced by known methods. The phthalic acid compounds may be commercially available products.

Among the phthalic acid compounds, acid dichloride (1) can be, for example, produced by reacting commercially available phthalic acid, isophthalic acid or terephthalic acid with a chlorinating agent such as thionyl chloride or phosphorus oxychloride (See e.g. Japanese Unexamined Patent Application Publication No. S61-109751). An acid anhydride can be, for example, obtained by heating an acid dichloride (1) in the presence of ethyl chlorofluoroacetate and chlorosulfonic acid (See e.g. J. Org. Chem., Volume 44, p. 2291 (1979)).

Chlorine gas is generally employed as chlorine. Chlorine may be commercially available chlorine gas and may be also chlorine gas produced by a known method (See e.g. Japanese Unexamined Patent Application Publication No. 2006-219369).

In the above reaction of a phthalic acid compound and chlorine, an amount of chlorine varies depending on the desired number of chlorine atoms which are substituted on the benzene ring (hereinafter the number is referred to as n).

The above chlorine is used in an amount of preferably n to 6n mole, more preferably n to 4n mole, and further preferably n mole or more and under 2n mole per mole of a phthalic acid compound.

In the above reaction of a phthalic acid compound and chlorine, unreacted chlorine can be reused for the reaction after recovered and, if needed, purified.

The above reaction of a phthalic acid compound and chlorine is carried out in the presence of an iodine compound.

The above iodine compound is not restricted as long as it is a compound containing an iodine atom, and the examples thereof include iodine chloride (ICl), iodine bromide (IBr) and iodine (I₂).

As the above iodine compound, a commercially available product can be employed. The above iodine compound is preferably iodine in view of availability.

The amount to be used of the above iodine compound is in the range of preferably 0.001 to 0.1 mole, and more preferably 0.005 to 0.05 mole per mole of a phthalic acid compound.

The above reaction of a phthalic acid compound and chlorine is carried out in a mixture of chlorosulfonic acid and thionyl chloride.

As the above chlorosulfonic acid, a commercially available product can be employed.

The amount to be used of chlorosulfonic acid is generally 0.5 parts by mass or more per part by mass of a phthalic acid compound. The upper limit is not particularly determined, and is preferably 20 parts by mass or less per part by mass of a phthalic acid compound in terms of productivity and the like.

As thionyl chloride, a commercially available product can be employed.

The amount to be used of thionyl chloride is generally 0.5 parts by mass or more per part by mass of a phthalic acid compound. The upper limit is not particularly determined, and is preferably 20 parts by mass or less per part by mass of a phthalic acid compound in terms of productivity and the like.

The above mixture of chlorosulfonic acid and thionyl chloride may contain chlorosulfonic acid and thionyl chloride, and a solvent which is inactive to the reaction. The solvent which is inactive to the reaction includes halogen solvents such as carbon tetrachloride and chloroform.

In the above reaction, the reaction temperature is preferably 0°C to 100°C, and more preferably 20°C to 80°C.

In the reaction of a phthalic acid compound and chlorine, the order of mixing the phthalic acid compound, chlorine, an iodine compound, chlorosulfonic acid and thionyl chloride is not restricted. A preferred embodiment includes an aspect of mixing a phthalic acid compound, an iodine compound, chlorosulfonic acid and thionyl chloride in any order, adjusting the temperature of the resulting mixture to the reaction temperature, and adding chlorine gas to the mixture. The addition of chlorine gas herein can be performed by blowing chlorine gas into a liquid phase in the reaction system; can be performed by circulating chlorine gas through a gas phase; and can be performed by replacing the gas phase with chlorine gas and, if needed, applying pressure.

The termination of the reaction is accordingly confirmed by general analytical means such as gas chromatography, high performance liquid chromatography, thin layer chromatography, NMR and IR.

A phthalic acid compound whose aromatic ring has been chlorinated is produced by the above reaction of a phthalic acid compound and chlorine (hereinafter this compound is referred to as chlorinated phthalic acid compound).

The above chlorinated phthalic acid compound includes a phthaloyl monohalide whose benzene ring is bound by 1 to 4 chlorine atoms, an isophthaloyl monohalide whose benzene ring is bound by 1 to 4 chlorine atoms, a terephthaloyl monohalide whose benzene ring is bound by 1 to 4 carbon atoms, a phthaloyl dihalide whose benzene ring is bound by 1 to 4 chlorine atoms, an isophthaloyl dihalide whose benzene ring is bound by 1 to 4 carbon atoms, a terephthaloyl dihalide whose benzene ring is bound by 1 to 4 chlorine atoms, and phthalic anhydride whose benzene ring is bound by 1 to 4 chlorine atoms.

The phthaloyl monohalide whose benzene ring is bound by 1 to 4 chlorine atoms includes a chlorophthaloyl monohalide, a dichlorophthaloyl monohalide, a trichlorophthaloyl monohalide and a tetrachlorophthaloyl monohalide.

The isophthaloyl monohalide whose benzene ring is bound by 1 to 4 chlorine atoms includes a chloroisophthaloyl monohalide, a dichloroisophthaloyl monohalide, a trichloroisophthaloyl monohalide and a tetrachloroisophthaloyl monohalide.

The terephthaloyl monohalide whose benzene ring is bound by 1 to 4 chlorine atoms includes a chloroterephthaloyl monohalide, a dichloroterephthaloyl monohalide, a trichloroterephthaloyl monohalide and a tetrachloroterephthaloyl monohalide.

The phthaloyl dihalide whose benzene ring is bound by 1 to 4 chlorine atoms includes a chlorophthaloyl dihalide, a dichlorophthaloyl dihalide, a trichlorophthaloyl dihalide and a tetrachlorophthaloyl dihalide.

The isophthaloyl dihalide whose benzene ring is bound by 1 to 4 chlorine atoms includes a chloroisophthaloyl dihalide, a dichloroisophthaloyl dihalide, a trichloroisophthaloyl dihalide and a tetrachloroisophthaloyl dihalide.

The terephthaloyl dihalide whose benzene ring is bound by 1 to 4 chlorine atoms includes a chloroterephthaloyl dihalide, a dichloroterephthaloyl dihalide, a trichloroterephthaloyl dihalide and a tetrachloroterephthaloyl dihalide.

In each of the acid monohalides and acid dihalides exemplified above, haloformyl groups include a fluoroformyl group, a chloroformyl group, an iodoformyl group and a bromoformyl group.

In the reaction of a phthalic acid compound and chlorine, for example, when the above acid dichloride (1) is used as a phthalic acid compound, a chlorinated phthalic acid compound represented by formula (2) 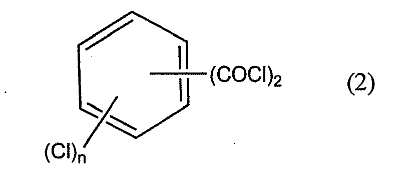 (wherein n is an integer of from 1 to 4) is obtained.

In the above reaction, when a compound containing a carboxy group is used as a phthalic acid compound, an acid monohalide or an acid dihalide is obtained as a chlorinated phthalic acid compound. When phthalic acid, isophthalic acid or terephthalic acid is, for example, used as a phthalic acid compound, an acid monohalide or an acid dihalide is obtained as a chlorinated phthalic acid compound. When an acid monohalide of phthalic acid, isophthalic acid or terephthalic acid is used as a phthalic acid compound, an acid dihalide is obtained as a chlorinated phthalic acid compound.

In the above chlorinated phthalic acid compounds, the benzene ring may be bound by 1 to 3 substituents such as a halogen atom, a nitro group, a sulfo group and a trihalomethyl group like the above phthalic acid compounds.

Among the above chlorinated phthalic acid compounds, a compound which has an unsubstituted position on the benzene ring can be used as a phthalic acid compound for the reaction of a phthalic acid compound and chlorine.

The above chlorinated phthalic acid compounds are preferably a phthaloyl monohalide whose benzene ring is bound by 1 to 4 chlorine atoms, an isophthaloyl monohalide whose benzene ring is bound by 1 to 4 chlorine atoms, a terephthaloyl monohalide whose benzene ring is bound by 1 to 4 chlorine atoms, a phthaloyl dihalide whose benzene ring is bound by 1 to 4 chlorine atoms, an isophthaloyl dihalide whose benzene ring is bound by 1 to 4 chlorine atoms, and a terephthaloyl dihalide whose benzene ring is bound by 1 to 4 chlorine atoms; more preferably a phthaloyl dihalide whose benzene ring is bound by 1 to 4 chlorine atoms, an isophthaloyl dihalide whose benzene ring is bound by 1 to 4 chlorine atoms and a terephthaloyl dihalide whose benzene ring is bound by 1 to 4 chlorine atoms; further preferably an isophthaloyl dihalide whose benzene ring is bound by 1 to 4 chlorine atoms and a terephthaloyl dihalide whose benzene ring is bound by 1 to 4 chlorine atoms; and particularly preferably a tetrachloroisophthaloyl dihalide and a tetrachloroterephthaloyl dihalide. When the above chlorinated phthalic acid compounds are represented by formula (2), a compound wherein n is 4 in formula (2) is preferred.

A chlorinated phthalic acid compound can be isolated from the reaction mixture obtained from the above reaction of a phthalic acid compound and chlorine by a conventional means. When a chlorinated phthalic acid compound is, for example, a chlorinated phthalic acid compound whose benzene ring is bound by 4 chlorine atoms, the chlorinated phthalic acid compound can be isolated from the reaction mixture as a solid by performing filtration, decantation and the like. The above reaction mixture may be, if needed, concentrated before a chlorinated phthalic acid compound is isolated.

When the above chlorinated phthalic acid compound isolated from the reaction mixture obtained from the reaction of a phthalic acid compound and chlorine is a solid, it is preferably washed with a solvent such as chlorosulfonic acid or thionyl chloride, and dried. Further, the above chlorinated phthalic acid compound isolated from the reaction mixture may be purified by conventional purifying means such as recrystallization and column chromatography.

A mixture containing chlorosulfonic acid, thionyl chloride, an iodine compound and the like is obtained by isolating a chlorinated phthalic acid compound from the above reaction mixture obtained from the reaction of a phthalic acid compound and chlorine. This mixture can be employed for the above reaction of a phthalic acid compound and chlorine. When the mixture is used for the above reaction of a phthalic acid compound and chlorine, a phthalic acid compound and chlorine as well as, if needed, an iodine compound, chlorosulfonic acid and/or thionyl chloride can be added to the mixture. A method for producing a chlorinated phthalic acid compound, wherein the reaction of a phthalic acid compound and chlorine is carried out in the presence of the above mixture containing chlorosulfonic acid, thionyl chloride and an iodine compound, is also one of the present invention.

A method for producing a chlorinated phthalic acid compound, the method comprising steps of: reacting a phthalic acid compound and chlorine in the presence of an iodine compound in a mixture of chlorosulfonic acid and thionyl chloride to obtain a reaction mixture; and isolating a mixture containing chlorosulfonic acid, thionyl chloride and an iodine compound from the above reaction mixture to obtain the chlorinated phthalic acid compound, is also one of the present invention.

In the above method, each step can be carried out under the same conditions as those described above. In the above method, the above chlorinated phthalic acid compound isolated from the reaction mixture is preferably a compound represented by formula (2), and more preferably a compound represented by formula (2) wherein n is 4.

In the present method, the reaction of a phthalic acid compound and chlorine is preferably carried out in the presence of a mixture containing chlorosulfonic acid, thionyl chloride and an iodine compound.

The above reaction of a phthalic acid compound and chlorine may be carried out by adding a phthalic acid compound and chlorine to the mixture containing chlorosulfonic acid, thionyl chloride and an iodine compound in unchanged form, and may be carried out by adding chlorosulfonic acid, thionyl chloride and/or an iodine compound to the mixture and further adding a phthalic acid compound and chlorine.

### EXAMPLES

The present invention will now be described in more detail by way of examples thereof.

### Example 1

In a 500 mL flask equipped with a reflux condenser and a chlorine inlet tube, 30 g of terephthaloyl dichloride, 105 g of chlorosulfonic acid, 148 g of thionyl chloride and 1.5 g of iodine were charged, and the resulting mixed liquid was heated to 60°C. Chlorine gas was blown into this mixed liquid at a flow rate of 30 to 50 ml/min for 10 hours. The amount used of chlorine gas was 84.3 g. Crystals were deposited in the reaction mixture. The reaction mixture was filtered, and the crystals obtained on the filter paper were washed with 40 g of thionyl chloride, and dried with nitrogen (normal temperature, 25°C) to yield 35 g of crystals containing 2,3,5,6-tetrachloroterephthaloyl dichloride.

When the resulting crystals were measured by gas chromatography (internal standard method), the content of 2, 3, 5, 6-tetrachloroterephthaloyl dichloride was 83. 7% by weight and the yield thereof was 58%. The other components contained in the crystals were mainly thionyl chloride, and unreacted terephthaloyl dichloride and a compound whose benzene ring was bound by 1 to 3 chlorine atoms were not detected.

The filtrate obtained by filtering the reaction mixture, 259 g, was recovered and the filtrate in unchanged form was used in Example 2.

### Example 2

In a 500 mL flask equipped with a reflux condenser and a chlorine inlet tube, 30 g of terephthaloyl dichloride, 259 g of the filtrate obtained in Example 1 and 0. 5 g of iodine were charged, and the resulting mixed liquid was heated to 60°C. Chlorine gas was blown into the mixed liquid at a flow rate of 20 to 40 ml/min for 10 hours. The amount used of chlorine gas was 69 g. Crystals were deposited in the reaction mixture. The reaction mixture was filtered, and the crystals obtained on the filter paper were washed with 40 g of thionyl chloride, and dried with nitrogen (normal temperature, 25°C) to yield 35.1 g of crystals containing 2,3,5,6-tetrachloroterephthaloyl dichloride. When the resulting crystals were measured by gas chromatography (internal standard method), the content of 2,3,5,6-tetrachloroterephthaloyl dichloride was 95. 4% by weight and the yield thereof was 66% (on the basis of terephthaloyl dichloride charged in Example 2 (30 g)). The other components contained in the crystals were mainly thionyl chloride, and unreacted terephthaloyl dichloride and a compound whose benzene ring was bound by 1 to 3 chlorine atoms were not detected. The filtrate obtained by filtering the reaction mixture, 245 g, was recovered and the filtrate in unchanged form was used in Example 3.

### Example 3

In a 500 mL flask equipped with a reflux condenser and a chlorine inlet tube, 30 g of terephthaloyl dichloride, 245 g of the filtrate obtained in Example 2 and 0. 5 g of iodine were charged, and the resulting mixed liquid was heated to 60°C. Chlorine gas was blown into the mixed liquid at a flow rate of 20 to 40 ml/min for 10 hours. The amount used of chlorine gas was 69 g. Crystals were deposited in the reaction mixture. The reaction mixture was filtered, and the crystals obtained on the filter paper were washed with 40 g of thionyl chloride, and dried with nitrogen (normal temperature, 25°C) to yield 58.3 g of crystals containing 2,3,5,6-tetrachloroterephthaloyl dichloride. When the resulting crystals were measured by gas chromatography (internal standard method), the content of 2,3,5, 6-tetrachloroterephthaloyl dichloride was 76.4% by weight and the yield thereof was 88% (on the basis of terephthaloyl dichloride charged in Example 3 (30 g)). The other components contained in the crystals were mainly thionyl chloride, and unreacted terephthaloyl dichloride and a compound whose benzene ring was bound by 1 to 2 chlorine atoms were not detected, and a compound whose benzene ring was bound by 3 chlorine atoms was 0.3% by weight of the crystals. The filtrate obtained by filtering the reaction mixture, 245 g, was recovered and the filtrate in unchanged form was used in Example 4.

### Example 4

In a 500 mL flask equipped with a reflux condenser and a chlorine inlet tube, 30 g of terephthaloyl dichloride, 245 g of the filtrate obtained in Example 3 and 0. 5 g of iodine were charged, and the resulting mixed liquid was heated to 60°C. Chlorine gas was blown into this mixed liquid at a flow rate of 20 to 40 ml/min for 10 hours. The amount used of chlorine gas was 69 g. Crystals were deposited in the reaction mixture. The reaction mixture was filtered, and the crystals obtained on the filter paper were washed with 40 g of thionyl chloride, and dried with nitrogen (normal temperature, 25°C) to yield 56.1 g of crystals containing 2,3,5,6-tetrachloroterephthaloyl dichloride. When the resulting crystals were measured by gas chromatography (internal standard method), the content of 2,3,5, 6-tetrachloroterephthaloyl dichloride was 83.8% by weight, and the yield thereof was 93% (on the basis of terephthaloyl dichloride charged in Example 4 (30 g)). Unreacted terephthaloyl dichloride and a compound whose benzene ring was bound by 1 to 2 chlorine atoms were not detected and a compound whose benzene ring was bound by 3 chlorine atoms was 0.4% by weight of the crystals. The filtrate obtained by filtering the reaction mixture, 240 g, was recovered and the filtrate in unchanged form was used in Example 5.

### Example 5

In a 500 mL flask equipped with a reflux condenser and a chlorine inlet tube, 30 g of terephthaloyl dichloride, 240 g of the filtrate obtained in Example 4 and 0.5 g of iodine were charged, and the resulting mixed liquid was heated to 60°C. Chlorine gas was blown into this mixed liquid at a flow rate of 20 to 40 ml/min for 10 hours. The amount used of chlorine gas was 69 g. Crystals were deposited in the reaction mixture. The reaction mixture was filtered, and the crystals obtained on the filter paper were washed with 40 g of thionyl chloride, and dried with nitrogen (normal temperature, 25°C) to yield 60.1 g of crystals containing 2,3,5,6-tetrachloroterephthaloyl dichloride. When the resulting crystals were measured by gas chromatography (internal standard method), the content of 2,3,5,6-tetrachloroterephthaloyl dichloride was 80.4% by weight, and the yield thereof was 96% (on the basis of terephthaloyl dichloride charged in Example 5 (30 g)). Unreacted terephthaloyl dichloride and a compound whose benzene ring was bound by 1 to 2 chlorine atoms were not detected and a compound whose benzene ring was bound by 3 chlorine atoms was 0.9% by weight of the crystals.

The total yield of 2,3,5,6-tetrachloroterephthaloyl dichloride obtained in Examples 1 to 5 [(the total number of moles of 2,3,5,6-tetrachloroterephthaloyl dichloride obtained in Examples 1 to 5) / (the total number of moles of terephthaloyl dichloride used in Examples 1 to 5) x 100] was 80%.

### Example 6

In a 200 mL flask equipped with a reflux condenser and a chlorine inlet tube, 10 g of terephthaloyl dichloride, 52 g of chlorosulfonic acid, 50 g of thionyl chloride and 0.5 g of iodine were charged, and the resulting mixed liquid was adjusted to 25°C. Chlorine gas was blown into this mixed liquid at a flow rate of 18 ml/min for 1 hour. The amount used of chlorine gas was 3.5 g. When the resulting reaction mixture was analyzed by gas chromatography (area percentage method), the results were as follows.
Terephthaloyl dichloride: 38.6%
2-Chloroterephthaloyl dichloride: 11.0%
2,3-Dichloroterephthaloyl dichloride,
2,5-dichloroterephthaloyl dichloride and
2,6-dichloroterephthaloyl dichloride: 48.0% (the total of 3 compounds)
2,3,5-Trichoroterephthaloyl dichloride: 2.4%

### Comparative Example 1

The same reactions as in Example 6 were repeated except that 50 g of carbon tetrachloride was used in place of thionyl chloride, and when the resulting reaction mixture was analyzed, the results were as follows.
Terephthaloyl dichloride: 75.9%
2-Chloroterephthaloyl dichloride: 11.8%
2,3-Dichloroterephthaloyl dichloride,
2,5-dichloroterephthaloyl dichloride and
2,6-dichloroterephthaloyl dichloride: 12.4% (the total of 3 compounds)
2,3,5-Trichoroterephthaloyl dichloride: 0.0%

### INDUSTRIAL APPLICABILITY

The present invention is useful as a method for producing a phthalic acid compound whose aromatic ring has been chlorinated.

## Claims

1. A method for producing a phthalic acid compound whose aromatic ring has been chlorinated, said method comprising reacting a phthalic acid compound and chlorine in the presence of an iodine compound in a mixture of chlorosulfonic acid and thionyl chloride.

2. The production method according to claim 1, wherein the phthalic acid compound is phthalic acid, isophthalic acid, terephthalic acid, a phthaloyl monohalide, an isophthaloyl monohalide, a terephthaloyl monohalide, a phthaloyl dihalide, an isophthaloyl dihalide or a terephthaloyl dihalide.

3. The production method according to claim 1, wherein the phthalic acid compound is phthalic acid, a phthaloyl dihalide, isophthalic acid, an isophthaloyl dihalide, terephthalic acid or a terephthaloyl dihalide.

4. The production method according to claim 1, wherein the phthalic acid compound is represented by formula (1) 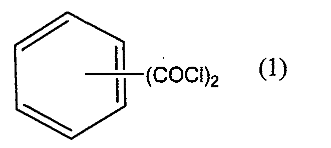 and the phthalic acid compound whose aromatic ring has been chlorinated is represented by formula (2) 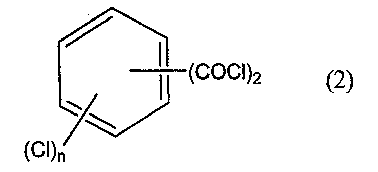 wherein n is an integer of from 1 to 4.

5. The production method according to claim 4, wherein n is 4 in formula (2).

6. The production method according to claim 1, wherein the iodine compound is iodine, iodine bromide or iodine chloride.

7. A method for producing a phthalic acid compound whose aromatic ring has been chlorinated, the method comprising steps of:
reacting a phthalic acid compound and chlorine in the presence of an iodine compound in a mixture of chlorosulfonic acid and thionyl chloride to obtain a reaction mixture; and
isolating a mixture containing chlorosulfonic acid, thionyl chloride and the iodine compound from said reaction mixture to obtain the phthalic acid compound whose aromatic ring has been chlorinated.

8. The production method according to claim 7, wherein the phthalic acid compound whose aromatic ring has been chlorinated is represented by formula (2) 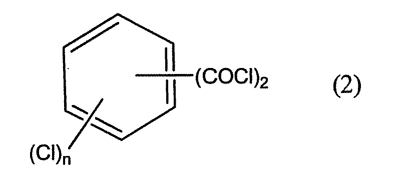 wherein n is an integer of from 1 to 4.

9. The production method according to claim 8, wherein n is 4 in formula (2).

10. A method for producing a phthalic acid compound whose the aromatic ring has been chlorinated, wherein a reaction of a phthalic acid compound and chlorine is performed in the presence of the mixture described in claim 7.

11. A method for producing a phthalic acid compound whose aromatic ring has been chlorinated, the method comprising steps of:
reacting a phthalic acid compound and chlorine in the presence of an iodine compound in a mixture of chlorosulfonic acid and thionyl chloride to obtain a reaction mixture;
isolating a mixture containing chlorosulfonic acid, thionyl chloride and the iodine compound from said reaction mixture to obtain the phthalic acid compound whose aromatic ring has been chlorinated; and
performing a reaction of a phthalic acid compound and chlorine in the presence of said mixture.
